# EUROPÄISCHE PATENTSCHRIFT

(11) **EP 1 660 622 B1**
(45) Veröffentlichungstag und Bekanntmachung des Hinweises auf die Patenterteilung: **30.09.2009**
(21) Anmeldenummer: 04764447.1
(22) Anmeldetag: 25.08.2004
(51) Int. Cl.: C11D 17/00

(54) **MEHRPHASIGES TENSIDPRODUKT**
MULTIPHASE SURFACTANT PRODUCT
PRODUIT TENSIOACTIF COMPRENANT PLUSIEURS PHASES

(30) Priorität: 03.09.2003 DE 10341024
(43) Veröffentlichungstag der Anmeldung: 31.05.2006
(73) Patentinhaber: Henkel AG & Co. KGaA, 40589 Düsseldorf (DE)
(72) Erfinder: JUNG, Lars, 40476 Düsseldorf (DE)
(86) Internationale Anmeldenummer: PCT/EP2004/009470
(87) Internationale Veröffentlichungsnummer: WO 2005/023973

(56) Entgegenhaltungen:
- WO-A-00/61716
- DE-A- 19 951 635
- US-A1- 2003 003 069

## Beschreibung

Die vorliegende Erfindung betrifft eine mindestens zwei visuell getrennte wässrige Phasen umfassende Zubereitung sowie ihre Verwendung als kosmetisches Haut- oder Haarbehandlungsmittel.

In der letzten Zeit hat es nicht an Bemühungen gefehlt mehrphasige Zubereitungen herzustellen, deren Phasen im Ruhezustand entmischt vorliegen und bei Bewegung kurzzeitig ineinander dispergierbar sind. Sie sollen, insbesondere bei Verwendung für kosmetische Zwecke, den Produkten ein attraktives Aussehen verleihen und ihre Wirkung für den Verbraucher visualisieren.

So werden in DE 161 72 08 A1 flüssige Reinigungsmittel beschrieben, die eine wässrige und eine ölige Phase umfassen und durch Schütteln kurzzeitig in eine Öl-in-Wasser-Emulsion überführt werden können. Auch in US 3,718,609 oder US 3,810,478 werden zweiphasige Shampoozusammensetzungen auf der Basis einer Ölphase und einer Wasserphase offenbart, die kurz vor der Anwendung durch Schütteln in eine instabile Emulsion überführt werden.

Die Herstellung solcher Emulsionen erfordert jedoch einen hohen Ölgehalt, was für viele Anwendungszwecke nachteilig ist. Beispielsweise eignen sich solche Emulsionen nicht für kosmetische Reinigungsprodukte, da sie einen fettigen Film auf Haut- oder Haaren hinterlassen würden, was für den Anwender unangenehm wäre.

Deshalb wurden Zweiphasenpräparate auf der Basis zweier kurzzeitig mischbarer wässriger Phasen vorgeschlagen:

Die Zusammensetzungen gemäß EP 116 422 und EP 175 485 umfassen zur Einstellung der Zweiphasigkeit mindestens 6 Gew.-% Komplexbildner, insbesondere Natriumhexametaphosphat. Ein solch hoher Gehalt an kondensierten Phosphaten ist aber weder anwendungstechnisch vorteilhaft, noch ökonomisch erwünscht.

In WO 00/61716 wird eine auf zwei entmischten wässrigen Phasen basierende Zubereitung vorgeschlagen, die eine oberFlächenaktive Verbindung, eine gelöste Pölymerverbindung und zur rascheren Entmischung der beiden Phasen einen ein- oder mehrwertigen Alkohol mit 2 bis 9 C-Atomen, vorzugsweise Ethanol oder Isopropanol, enthält. Dabei handelt es sich aber um dünnflüssige Formulierungen, die in der Handhabung als Dusch- oder Shampooprodukt schwierig sind, da sie schnell von der Hand rinnen. Dies führt dazu, dass der größte Teil des Produktes bei der Anwendung ungenutzt verloren geht.
Darüber hinaus trocknen Alkohole wie Ethanol und Isopropanol die Haut aus.

Die WO 02/15849 beschreibt zweiphasige Reinigungsmittel für Haut- und Haar, die Tenside, Verdickungsmittel, Polyalkylenglycole und nicht-komplexierende Mineralsalze enthalten. Die beiden wässrigen Phasen lassen sich durch Schütteln vermischen und trennen sich innerhalb von 24 Stunden wieder in ein Zweiphasenprodukt auf. Nachteilig an diesen Mitteln ist, dass die Polyalkylenglycole zwingend sind, da sie eine Ausflockung der Tenside in der Tensidphase verhindern sollen. Der Einsatz von Polyethylenglycolen in kosmetischen Präparaten wird aber in der Öffentlichkeit kontrovers diskutiert, da sie aufgrund ihrer penetrationsfördernden Eigenschaften in der Presse (Öko-Test) negativ bewertet wurden. Darüber hinaus sind die Entmischungszeiten der Zweiphasenpräparate gemäß WO 02/15849 sehr langsam.

Aufgabe der vorliegenden Erfindung war daher eine zweiphasige Zubereitung herzustellen, bei der eine rasche Vermischung und Entmischung der beiden wässrigen Phasen gewährleistet wird. Die Zubereitungen sollen sich für die Verwendung als kosmetisches Mittel eignen und insbesondere als Hautbehandlungsmittel bedingt durch physiologisch unbedenkliche Inhaltsstoffe für ein angenehmes Hautgefühl, eine gute Hautverträglichkeit und eine feuchtigkeitsspendende Wirkung sorgen. Darüber hinaus sollen die Zubereitungen stabil sein, d.h., dass die Ausflockung der Tenside in der Tensidphase verhindert werden soll.

Diese Aufgabe wurde überraschenderweise durch die Kombination spezieller Komponenten in einem wässrigen Zweiphasenpräparat in hohem Maße erfüllt.

Gegenstand der vorliegenden Erfindung sind daher Zubereitungen auf der Basis zweier im Ruhezustand visuell voneinander entmischten, wässrigen Phasen, die durch Bewegung kurzzeitig ineinander dispergierbar sind und
- 0,1 bis 30 Gew.-% mindestens eines Tensids
- 10 bis 25 Gew.-% mindestens eines Salzes ausgewählt aus Natrium-, Calcium- oder Magnesiumchlorid, Natriumcarbonat- oder -hydrogencarbonat, Natrium- oder Magnesiumsulfat, Natriumhydrogenphosphat, Natriumacetat, -citrat, -lactat, -benzoat, -tartrat,-succinat oder einem Gemisch dieser Salze und
- 5 bis 25 Gew.-% mindestens eines C₃-C₁₂-Alkohols mit wenigstens 3 OH-Gruppen im Molekül,
enthalten.

Die Gehaltsangaben beziehen sich, sofern nicht anders angegeben, jeweils auf das Gesamtgewicht der Zubereitungen.

Gemäß einer bevorzugten Ausführungsform der Erfindung werden die beiden wässrigen Phasen durch Schütteln ineinander dispergiert. Die reversible Entmischung in zwei Phasen erfolgt bevorzugt durch einfaches Stehenlassen der Zubereitungen nach der Durchmischung. Eine erste sichtbare Trennung der Phasen tritt bereits nach etwa 10 min auf. Die vollständige Entmischung ist nach maximal 12 Stunden abgeschlossen.

Die beiden wässrigen Phasen sind vor und nach der Durchmischung klar; eine Ausflockung der Tenside konnte nicht beobachtet werden.

Die erfindungsgemäßen Zubereitungen hinterlassen bei der Hautbehandlung ein angenehmes, nicht fettiges Hautgefühl. Ferner zeichnen sich die erfindungsgemäßen Zubereitungen durch eine gute Hautverträglichkeit aus, insbesondere da auf den Einsatz von Polyethylenglycolen verzichtet werden kann.

Gemäß einer bevorzugten Ausführungsform der Erfindung enthalten die Zubereitungen mindestens ein anionisches Tensid.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30C-Atomen. Zusätzlich können im Molekül Glykol- oder Polyglykolether-Gruppen, Ester-, Ether und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte - Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppel bindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (I), 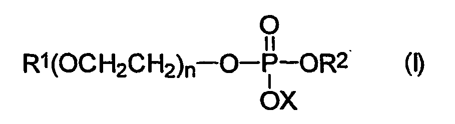 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für einen C₁ bis C₄- Kohlenwasserstoffrest, steht,
- sulfatierte Fettsäurealkylenglykolester der Formel (II)

   R⁷CO(AlkO)ₙSO₃M Formel (II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (III) wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind. 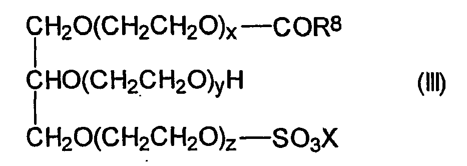 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (III) eingesetzt, in der R⁸CO für einen linearen Acylrest mit 8 bis 18 Kohlenstoffatomen steht.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

In einer bevorzugten Ausführungsform der Erfindung beträgt der Gehalt an Aniontensiden 15-20 Gew.-%.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acylaminopropyl-N,N-dimethylammoniumglycinate, beispielsweise das Kokosacylaminopropyl-dimethylammoniumglycinat, und 2Alkyl-3-carboxymethyl-3-hydroxyethyl-imidazoline mit jeweils 8 bis 18 CAtomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - A-cylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propyl lenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (IV)

   R⁹CO-(OCH₂CHR¹⁰)_{w}OR¹¹ Formel (IV),

   in der R⁹CO für einen linearen oder verzweigten, gesättigten und/oder ungesättigten Acylrest mit 6 bis 22 Kohlenstoffatomen, R¹⁰ für Wasserstoff oder Methyl, R¹¹ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettaminie,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₁-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die mologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Als weitere Tenside werden gemäß einer bevorzugten Ausführungsform der Erfindung amphotere und/oder zwitterionische Tenside eingesetzt.

Die weiteren Tenside werden in Mengen von 1-10 Gew.%, bevorzugt 2-6 Gew.% und ganz besonders bevorzugt von 3-5 Gew.%, bezogen auf das gesamte Mittel, eingesetzt.

Als weitere Komponente ist in den erfindungsgemäßen Zubereitungen mindestens ein Salz enthalten.

Das Salz wird ausgewählt aus Natrium-, Calcium- oder Magnesiumchlorid, Natriumcarbonat- der - hydrogencarbonat, Natrium- oder Magnesiumsulfat, Natriumhydrogenphosphat, Natriumacetat, -lactat, -citrat, -benzoat -tartrat oder -succinat, sowie aus Gemischen dieser Salze.

Das Salz oder Salzgemisch ist in den erfindungsgemäßen Zubereitungen üblicherweise in einer Menge von 10 bis 25 Gew.-%, bevorzugt in einer Menge von 12. bis 22 Gew.-% und insbesondere in einer Menge von 15 bis 20 Gew.-% enthalten.

Die erfindungsgemäßen Zubereitungen enthalten weiterhin mindestens einen C₃-C₁₂-Alkohol mit wenigstens 3 OH-Gruppen im Molekül. Diese sind vorzugsweise ausgewählt aus Glycerin, Erythrit, Diglycerin, Triglycerin, C₅- und C₆-Zuckeralkoholen wie Arabit, Xylit, Galactit, Mannit und Sorbit, Inosit und Zuckern wie Sucrose.

Erfindungsgemäß besonders geeignete C₃-C₁₂-Alkohole sind Glycerin, Diglycerin, Triglycerin, Sorbit und Sucrose, insbesondere geeignet ist Sorbit.

Gemäß einer weiteren bevorzugten Ausführungsform der Erfindung enthalten die Zubereitungen weiterhin mindestens ein Verdickungsmittel. Dieses ist üblicherweise ausgewählt aus Agar-Agar, Guar-Gum, Alginaten, Xanthan-Gum, Gummi arabicum, Karaya-Gum, Johannisbrotkernmehl, Leinsamengummen, Dextranen, Cellulose-Derivaten wie Methylcellolose, Hydroxyalkylcellulose und Carboxymethylcellulose, Stärke-Fraktionen und Derivaten wie Amylose, Amylopektin und Dextrinen, sowie Tonen wie Bentonit. Insbesondere bevorzugte Verdickungsmittel im Sinne der Erfindung sind die unter den INCI-Bezeichnungen Polyether-1 (Handelsnamen: Pure Thix 1442; Südchemie), PEG-200 Hydrogenated Glyceryl Palmate (Rewoderm LI S 80) oder REWODERM LI 520-70;Degussa Goldschmidt), PEG-120 Methyl Glucose Dioleate (Glucamate DOE-120; Amerchol) erhältlichen Produkte sowie Xanthan Gum und alle hydrophob modifizierten Polyalkylenglycole.

Die erfindungsgemäßen Zubereitungen weisen eine Viskosität von mindestens 400 mPa*s auf (gemessen bei 20°C mit einem Brookfield DV-II+ Viskosimeter; Spindel RV3, 20 min⁻¹ Messdauer 1 min).

Üblicherweise steht eine höhere Viskosität einer schnellen Entmischung der Phasen aus physikalischen Gründen entgegen (Stokes-Einstein-Beziehung). In den erfindungsgemäßen Zubereitungen wird aufgrund der hohen Dichteunterschiede zwischen den Phasen der Einfluss der Viskosität auf die Sinkgeschwindigkeit allerdings zurückgedrängt, deshalb findet ebenfalls eine schnelle Entmischung statt (im Vergleich zu den Zubereitungen gemäß WO 00/61716). Vorteilhaft für die schnelle Entmischung ist auch die Verdrängung der Tenside durch das eingesetzte Salz aus der ursprünglich einphasigen Tensidlösung.

Neben den genannten Komponenten können die erfindungsgemäßen Zweiphasenzubereitungen auch weitere, für die Anwendung oder das Aussehen vorteilhafte Komponenten enthalten, wie sie in kosmetischen Haut- und Haarbehandlungsmitteln üblich sind.

Beispielsweise können die erfindungsgemäßen Zubereitungen in einer weiteren bevorzugten Ausführungsform Proteinhydrolysate und/oder Aminosäuren und deren Derivate (H) enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare. Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie ß-Alanin, Anthranilsäure oder Hippursäure eingesetzt werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) er hältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Die Proteinhydrolysate oder deren Derivate sind in den erfindungsgemäßen Zubereitungen bevorzugt in Mengen von 0,1 bis 10 Gew.%, bezogen auf das gesamte Mittel, enthalten. Mengen von 0,1 bis 5 Gew.-% sind besonders bevorzugt.

Ebenfalls als vorteilhaft hat sich die Verwendung von Vitaminen, Provitaminen und Vitaminvorstufen sowie deren Derivaten in den erfindungsgemäßen Zubereitungen erwiesen. Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
Vitamin B₁ (Thiamin).
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevor zugt in Mengen von 0,05 bis 1 Gew.%, bezogen auf das gesamte Mittel, enthalten ist.
Vitamin B_{5.} (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Pänthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1-5 Gew.-% sind besonders bevorzugt.
Vitamin B₆ (Pyridoxin sowie Pyridoxamin und Pyridoxal).

Vitamin C (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure; verstanden.

Vitamin H. Als Vitamin H wird die Verbindung (3a*S*,4*S*, 6a*R*)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.%, insbesondere in Mengen von 0,001 bis 0,01 Gew.-% enthalten.

Bevorzugt enthalten die erfindungsgemäß verwendeten Zubereitungen Vitamine, Provitamine und Vitaminvorstufen aus den Gruppen A, B, E und H. Selbstverständlich können auch mehrere Vitamine und Vitaminvorstufen gleichzeitig enthalten sein.

Panthenol, Pantolacton, Pyridoxin und seine Derivate sowie Nicotinsäureamid und Biotin sind besonders bevorzugt.

Die Einsatzmenge der Vitamine und Vitaminvorstufen in den erfindungsgemäßen Mitteln beträgt 0,0001 - 10 Gew.%, bezogen auf das gesamte Mittel, bevorzugt 0,0001 - 5 Ges.-%, und insbesondere 0,0001 - 3 Gew.%.

Schließlich können in den erfindungsgemäßen Mitteln Pflanzenextrakte verwendet werden. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmitteigemisch.

Weiterhin kann es bevorzugt sein, in den erfindungsgemäßen Mitteln Mischungen aus mehreren, insbesondere aus zwei, verschiedenen Pflanzenextrakten einzusetzen.

Die Einsatzmenge der Pflanzenextrakte in den erfindungsgemäß verwendeten Mitteln beträgt üblicherweise 0,01 - 50 Gew.%, bezogen auf das gesamte Mittel, bevorzugt 0,1 - 30 Gew.%, und insbesondere 0,1 - 20 Gew.-%.

In einer weiteren bevorzugten Ausführungsform der Erfindung werden den Zubereitungen zur weiteren Wirkungssteigerung kationische Polymere zugesetzt.

Bevorzugte kationische Polymere im Sinne der Erfindung sind beispielsweise kationische Cellulosederivate, wie z.B. eine quaternierte Hydroxyethylcellulose, die unter der Bezeichnung Polymer JR^{®} 400 von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine^{®}/Sandoz), Copolymere der Acrylamid mit Dimethyldiallyl-ammoniumchlorid (Merquat^{®} 550/Chemviron), Homopolymere des Dimethyldiallyl-ammoniumchlorids (Merquat^{®} 100), Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol.

Insbesondere bevorzugte kationische Polymere sind kationische Guar-Derivate, kationische Cellulose-Derivate, Homopolymere des Dimethyldiallyl-anmoniulnchlorids sowie Copolymere des Dimethyldiallylammoniumchlorids mit Acrylamid.

Die kationischen Polymere werden in den erfindungsgemäßen Zubereitungen bevorzugt in Mengen von 0,001 bis 5 Gew.-%, bevorzugt in Mengen von 0,01 bis 3 Gew.-% und insbesondere in Mengen von 0,05 bis 2 Gew.-% eingesetzt.

Bei den erfindungsgemäßen Zubereitungen handelt es sich bevorzugt um Haut- oder Haarbehandlungsmittel, insbesondere um Haut- oder Haarreinigungsmittel wie Duschbäder, Shampoos, Schaumbäder, Flüssigseifen, Make-up-Remover und Gesichtsreiniger, deshalb ist ein zweiter Gegenstand der Erfindung die Verwendung der erfindungsgemäßen Zubereitungen als kosmetisches Haut und Haarbehandlungsmittel.

In einer bevorzugten Ausführungsform der Erfindung werden den Zubereitungen ein oder mehrere Farbstoffe, bevorzugt solche, die in den entmischten, wässrigen Phasen eine unterschiedliche Löslichkeit besitzen, zugesetzt. Dadurch kann man den Zubereitungen ein besonders ansprechendes Aussehen verleihen. Geeignete Farbstoffe sind beispielsweise Blue 1 (Cl 42090), Green 3 (Cl 42053), Green 5 (Cl 61570), Green 8 (Cl 59040), Yellow 5 (Cl 19140), Yellow 6 (Cl 15985), Yellow 10 (CI 47005), Red 4 (Cl 14700), Red 33 (Cl 17200) und Red 40 (Cl 16035).

Die weiteren Wirk-, Hilfs- und Zusatzstoffe, die in den bevorzugten Ausführungsformen in untergeordneten Mengen eingesetzt werden, sind beispielsweise
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol, Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Antioxidantien,
- pH-Regulatoren wie beispielsweise Citronensäure oder Milchsäure,
- Konservierungsmittel wie beispielsweise Benzoesäure oder Salicylsäure.

Bezüglich weiterer fakultativer Komponenten sowie die eingesetzten Mengen dieser Komponenten wird ausdrücklich auf die dem Fachmann bekannten einschlägigen Handbücher, z. B. die Monographie von K. H. Schrader, Grundlagen und Rezepturen der Kosmetika, 2. Auflage, Hüthig Buch Verlag Heidelberg, 1989, verwiesen.

Die folgenden Beispiele sollen den Gegenstand der Erfindung näher erläutern, ohne ihn darauf zu beschränken:

### Beispiele

Es wurde das folgende erfindungsgemäße 2-Phasen-Duschgel hergestellt:

| Komponente | Gew.-% |
|---|---|
| Magnesiumsulfat x 7H₂O | 20 |
| Sorbit (70%) | 11,4 |
| Natriumlaurethsulfat (70%) | 22,8 |
| Cocamidopropylbetain (39%) | 7,9 |
| Panthenol 75L | 0,15 |
| Cetiol HE | 0,5 |
| Rewoderm LI 520-70 | 1,5 |
| Glucamate DOE-120 | 1,0 |
| Konservierungsmittel, Farbstoff, Säuerungsmittel, Parfum, Wasser | ad 100 |

Die Rezeptur erwies sich im Stabilitätstest über 12 Wochen bei -20°C, 0°C, 5°C, 35°C und 45°C Raumtemperatur als stabil.

## Patentansprüche

1. Zubereitungen auf der Basis zweier im Ruhestand visuell voneinander entmischten, wässrigen Phasen, die durch Bewegung kurzzeitig ineinander dispergierbar sind und
- 0,1 bis 30 Gew.-% mindestens eines Tensids,
- 10 bis 25 Gew.-% mindestens eines Salzes, ***ausgewählt aus Natrium-, Calcium- oder Magnesiumchlorid, Natriumcarbonat oder-hydrogencarbonat, Natrium- oder Magnesiumsulfat, Natriumhydrogenphosphat, Natriumacetat, -citrat, -lactat, -benzoat,-tartrat, -succinat oder einem Gemisch dieser Salze**,* und
- 5 bis 25 Gew.-% mindestens eines C3-C12-Alkohols mit wenigstens 3 OH-Gruppen im Molekül,
enthalten.

2. Zubereitungen nach Anspruch 1, **dadurch gekennzeichnet, dass** sie mindestens ein anionisches Tensid enthalten.

3. Zubereitungen nach einem der Ansprüche 1 oder 2, **dadurch gekennzeichnet, dass** sie zusätzlich mindestens ein weiteres Tensid, ausgewählt aus der Gruppe der amphoteren, zwitterionischen, nichtionischen oder kationischen Tenside enthalten.

4. Zubereitungen nach einem der Ansprüche ***1 bis 3**,* **dadurch gekennzeichnet, dass** der Alkohol ausgewählt ist aus Glycerin, Erythrit, Diglycerin, Triglycerin, C₅- und C₆-Zuckeralkoholen wie Arabit, Xylit, Mannit, Sorbit, Inosit und Zuckern wie Sucrose.

5. Zubereitungen nach Anspruch ***4***, **dadurch gekennzeichnet, dass** der Alkohol ausgewählt ist aus Glycerin, Diglycerin, Triglycerin, Sorbit, und Sucrose.

6. Zubereitungen nach einem der Ansprüche ***1 bis 5***, **dadurch gekennzeichnet, dass** sie zusätzlich ein Verdickungsmittel enthalten.

7. Zubereitungen nach einem der Ansprüche ***1 bis 6***, **dadurch gekennzeichnet, dass** sie eine Viskosität von mindestens 400 mPa*s (gemessen bei einer Temperatur von 20°C mit einem Brookfield DV-II+ Viskosimeter; Spindel RV3; 20 min⁻¹ Messdauer 1 min) aufweisen.

8. Zubereitungen nach einem der Ansprüche ***1 bis 7***, **dadurch gekennzeichnet, dass** sie mindestens einen weiteren Wirkstoff aus der Gruppe der kationischen Polymere, Proteinhydrolysate, Pflanzenextrakte und Vitamine enthalten.

9. Zubereitungen nach Anspruch ***8**,* **dadurch gekennzeichnet, dass** sie Panthenol enthalten.

10. Verwendung einer Zubereitung nach einem der vorhergehenden Ansprüche als kosmetisches Haut- und Haarbehandlungsmittel.

## Claims

1. Preparations based on two aqueous phases visually demixed from each other at rest, which are briefly dispersible into each other by motion, and containing
- 0.1 to 30% by weight of at least one surfactant,
- 10 to 25% by weight of at least one salt, *selected from sodium, calcium or magnesium chloride, sodium carbonate or hydrogencarbonate, sodium or magnesium sulfate, sodium hydrogenphosphate, sodium acetate, citrate, lactate, benzoate, tartrate, succinate or a mixture* of *these salts,* and
- 5 to 25% by weight of at least one C₃-C₁₂ alcohol with at least 3 OH groups in the molecule.

2. Preparations according to claim 1, **characterized in that** they contain at least one anionic surfactant.

3. Preparations according to any of claims 1 or 2, **characterized in that** they additionally contain at least one further surfactant selected from the group of amphoteric, zwitterionic, non-ionic or cationic surfactants.

4. Preparations according to any of claims *1 to 3,* **characterized in that** the alcohol is selected from glycerol, erythritol, diglycerol, triglycerol, C₅ and C₆ sugar alcohols such as arabitol, xylitol, mannitol, sorbitol, inositol and sugars such as sucrose.

5. Preparations according to claim *4*, **characterized in that** the alcohol is selected from glycerol, diglycerol, triglycerol, sorbitol, and sucrose.

6. Preparations according to any of claims *1 to 5,* **characterized in that** they additionally contain a thickener.

7. Preparations according to any of claims *1 to 6*, **characterized in that** they have a viscosity of at least 400 mPa*s (measured at a temperature of 20°C with a Brookefield DV-II+ viscosimeter; spindle RV3; 20 min⁻¹; measurement duration 1 min).

8. Preparations according to any of claims *1 to 7*, **characterized in that** they contain at least one further active substance from the group of cationic polymers, protein hydrolysates, plant extracts and vitamins.

9. Preparations according to claim *8*, **characterized in that** they contain panthenol.

10. The use of a preparation according to any of the preceding claims as a cosmetic skin and hair treatment agent.

## Revendications

1. Préparations à base de deux phases aqueuses dissociées l'une de l'autre à l'oeil nu à l'état de repos, que l'on peut faire s'interpénétrer par dispersion en peu de temps en les remuant et qui contiennent
- à concurrence de 0,1 à 30 % en poids, au moins un agent tensioactif ;
- à concurrence de 10 à 25 % en poids, au moins un sel choisi parmi le chlorure de sodium, de calcium ou de magnésium, le carbonate ou l'hydrogénocarbonate de sodium, le sulfate de sodium ou de magnésium, l'hydrogénophosphate de sodium, l'acétate, le citrate, le lactate, le benzoate, le tartrate, le succinate de sodium ou encore un mélange de ces sels, et
- à concurrence de 5 à 25 % en poids, au moins un alcool en C₃-C₁₂ dont la molécule contient au moins trois groupes OH.

2. Préparations selon la revendication 1, **caractérisées en ce qu'**elles contiennent au moins un agent tensioactif anionique.

3. Préparations selon la revendication 1 ou 2, **caractérisées en ce qu'**elles contiennent en outre au moins un agent tensioactif supplémentaire choisi parmi le groupe des agents tensioactifs amphotères, zwitterioniques, non ioniques ou cationiques.

4. Préparations selon l'une quelconque des revendications 1 à 3, **caractérisées en ce que** l'alcool est choisi parmi le glycérol, l'érythritol, le diglycérol, le triglycérol, des alcools de sucre en C₅ et C₆, tels que l'arabitol, le xylitol, le mannitol, le sorbitol, l'inositol, et des sucres tels que le saccharose.

5. Préparations selon la revendication 4, **caractérisées en ce que** l'alcool est choisi parmi le glycérol, le diglycérol, le triglycérol, le sorbitol et le saccharose.

6. Préparations selon l'une quelconque des revendications 1 à 5, **caractérisées en ce qu'**elles contiennent en outre un épaississant.

7. Préparations selon l'une quelconque des revendications 1 à 6, **caractérisées en ce qu'**elles présentent une viscosité d'au moins 400 mPa*s (mesurée à une température de 20 °C avec un viscosimètre de Brookfield DV-II+ ; broche RV3 ; 20 min⁻¹ ; durée de la mesure 1 minute).

8. Préparations selon l'une quelconque des revendications 1 à 7, **caractérisées en ce qu'**elles contiennent au moins une substance active supplémentaire choisie parmi le groupe des polymères cationiques, des hydrolysats de protéines, des extraits de plantes et des vitamines.

9. Préparations selon la revendication 8, **caractérisées en ce qu'**elles contiennent du panthénol.

10. Utilisation d'une préparation selon l'une quelconque des revendications précédentes à titre d'agent de traitement cosmétique de la peau et des cheveux.
